# EUROPEAN PATENT APPLICATION

(11) **EP 4 711 000 A1**
(43) Date of publication of application: **18.03.2026**
(21) Application number: 24306512.5
(22) Date of filing: 13.09.2024
(51) Int. Cl.: A61N 5/10

(54) **INDICATION-AGNOSTIC, VENDOR-AGNOSTIC, MEDIUM-AGNOSTIC AUTOMATED RADIOTHERAPY PLANNING SYSTEM**

(71) Applicant: TheraPanacea, 75004 Paris (FR)
(72) Inventor: DELASALLES, Edouard, 75004 Paris (FR); BUS, Norbert, 75004 Paris (FR); MENGIN, Elie, 75004 Paris (FR); VAUCLIN, Rémi, 75004 Paris (FR); UNGUN, Baris, 75004 Paris (FR)
(74) Representative: Icosa

(57) **Abstract**

The present invention relates to an automated radiotherapy planning system for generating a deliverable radiotherapy plan (31) for a patient, to be delivered by a predefined radiotherapy delivery system, wherein the system is configured to segment a target volume and one or more organs at risks in a 3D medical image of the patient, generate a prediction of a 3D volumetric dose distribution, generate said deliverable radiotherapy plan (31) and send the deliverable radiotherapy plan (31) to a predefined radiotherapy delivery system for final verification and execution.

## Description

### FIELD OF INVENTION

The present invention relates to radiotherapy treatment planning. Notably the present invention relates to a system and a method for automated radiotherapy treatment planning, using a 3D medical image of a patient.

### BACKGROUND OF INVENTION

Radiotherapy is a critical modality in the treatment of cancer, leveraging high-energy radiation to destroy malignant cells while sparing surrounding healthy tissues. The success of radiotherapy is heavily dependent on the precision and accuracy of treatment planning, which involves the careful delineation of target volumes-such as tumors-and organs at risk (OARs). The goal is to deliver the prescribed dose to the target while minimizing exposure to adjacent healthy tissues. Traditionally, this process is manual, requiring significant input from radiation oncologists, dosimetrists, and medical physicists.

The conventional approach to radiotherapy planning is time-consuming and prone to variability. Clinicians and physicists must manually segment the target volumes and OARs, design the beam arrangements, and iteratively adjust the treatment plan to achieve the desired dose distribution. This process can take several hours to days, depending on the complexity of the case. Furthermore, the manual nature of the process introduces potential inconsistencies, as the outcome can vary based on the individual clinician's expertise and experience. These variations can lead to suboptimal treatment plans that may compromise patient outcomes.

Moreover, the radiotherapy planning process is often tied to specific vendor systems, which limits the flexibility of treatment centers to adopt new technologies or integrate with existing systems. Each vendor's planning system may have unique interfaces, file formats, and algorithms, creating challenges in interoperability and data exchange. This vendor dependence can further exacerbate the inefficiencies in the planning process, as clinicians may need to adapt to different systems when dealing with patients treated on different machines.

The emergence of deep learning and artificial intelligence (AI) has provided opportunities to automate and enhance various aspects of medical imaging and treatment planning. In radiotherapy, AI-driven systems have shown promise in automating the segmentation of target volumes and OARs, a critical step that directly influences the quality of the treatment plan. However, existing AI solutions are often designed for specific indications or integrated with specific vendor systems, limiting their applicability across different treatment scenarios.

The present invention addresses these limitations by introducing an automated radiotherapy planning system that is both indication-agnostic and vendor-agnostic.

### SUMMARY

The present invention notably relates to automated radiotherapy planning system for generating a deliverable radiotherapy plan for a patient, to be delivered by a predefined radiotherapy delivery system, wherein the system comprises:
- at least one input configured to receive:
   ∘ at least one 3D medical image obtained from a medical imaging modality, said at least one 3D medical image comprising a representation of a target volume to be treated, with said deliverable radiotherapy plan, and one or more organs at risks of said patient;
   ∘ dose prescription parameters for said patient, said dose prescription parameters comprising at least one maximum dose prescription and minimum dose prescription for the target volume and at least one maximum dose prescription for each of the one or more organs at risks;
   ∘ clinical configurable parameters associated to said predefined radiotherapy delivery system and said medical imaging modality;
- at least one processor configured to:
   ∘ segment said target volume and one or more organs at risks in the 3D medical image, obtaining a segmented target volume and one or more segmented organs at risks;
   ∘ generate a prediction of a 3D volumetric dose distribution based on the 3D medical image, the segmented target volume and one or more segmented organs at risks, and by applying voxel-wise conditioning variables based on said dose prescription parameters;
   ∘ generate said deliverable radiotherapy plan with an optimization algorithm using said volumetric dose distribution, said clinical configurable parameters, segmented target volume and one or more segmented organs at risks and said at least one 3D medical image patient anatomy;
   ∘ send the deliverable radiotherapy plan to the predefined radiotherapy delivery system for final verification and execution.

The automated system also overcomes the challenges of vendor dependency by providing a unified platform that can interface with multiple vendor systems. This allows treatment centers to leverage their existing infrastructure while benefiting from the advanced capabilities of the invention. The result is a more efficient, consistent, and accurate radiotherapy planning process that can be applied across a wide range of clinical indications, ultimately enhancing patient care and treatment outcomes.

According to other advantageous aspects of the invention, the system comprises one or more of the features described in the following embodiments, taken alone or in any possible combination.

According to one embodiment, generate a prediction of a volumetric dose distribution comprises:
- obtain a 3D electronic density distribution from the at least one 3D medical image and a clinical configurable parameter associated to said medical imaging modality;
- obtain voxel-wise conditioning variables based on said dose prescription parameters and said segmented target volume and one or more segmented organs at risks, wherein said voxel-wise conditioning variables comprises first conditioning variables and second conditioning variables,
   each first conditioning variable being associated to a voxel of the at least one 3D medical image and representing a minimum value of dose to be delivered to the target volume or to one or more organs at risks represented in said voxel;
   each second conditioning variable being associated to a voxel of the at least one 3D medical image and representing a maximum value of dose to be delivered to the target volume or to one or more organs at risks represented in said voxel;
- feed said 3D electronic density distribution, said first conditioning variables and said second conditioning variables to a previously trained dose prediction model configured to provide as output the predicted volumetric dose distribution.

According to one embodiment, the first conditioning variables and the second conditioning variables being scaled by the maximum dose prescription comprising the greatest magnitude among the dose prescription parameters; and the predicted volumetric dose distribution obtained from the dose prediction model is unscaled using said maximum dose prescription

According to one embodiment, the dose prediction model architecture is a convolutional neural network, preferably a U-net.

According to one embodiment, generate said deliverable radiotherapy plan with an optimization algorithm comprises:
- define a first objective function on the base of said predicted volumetric dose distribution and said clinical configurable parameters, said first objective function comprising at least weighted voxel-wise penalties on deviations from said predicted volumetric dose distribution;
- define an initial set of configuration samples, each configuration sample comprising at least a control point, a multi-leaves collimator (MLC) aperture, a dose rate, a number of delivered monitor units and a gantry angle;
- initialize the optimization algorithm to generate, from said initial set of configuration samples, a current set of configuration samples,
- obtain an optimal configuration sample by performing iterative optimization of said current set of configuration samples, wherein the optimization algorithm iteratively calculates a current dose distribution resulting from the current set of configuration samples and modifies the current set of configuration samples in order to minimize the first objective function, until convergence

According to one embodiment, the first objective function further comprises at least one among: weighted penalties associated to over-dosing or under-dosing regions of interests relative to predefined clinically relevant thresholds, weighted penalties to discourage overly complex multi-leaves collimator aperture shapes and multi-leaves collimator leaf travel patterns, and weighted penalties to discourage variance across control points over the number of delivered monitor units.

According to one embodiment, the optimization algorithm is based on a Root Mean Square Propagation (RSMProp) algorithm modified to ensure hard constraints on decision variables by way of iteration-dependent augmentation of first objective function terms related to constraints on clinical configurable parameters associated to said predefined radiotherapy delivery system.

According to one embodiment, the initial set of configuration samples comprises an initial multi-leaves collimator (MLC) aperture for each control point that is calculated using a column generation method configured to evaluate impact of multi-leaves collimator (MLC) apertures over all the control points of the initial set of configuration samples.

According to one embodiment, generate said deliverable radiotherapy plan with an optimization algorithm further comprises:
- obtain a simulated volumetric dose distribution using a dose engine and the obtained optimal configuration sample;
- generate said deliverable radiotherapy plan by minimizing a second objective function obtained from said current dose distribution using said optimization algorithm, wherein said second objective function is defined as the first objective function further comprising a term that penalizes deviation between said simulated volumetric dose distribution and the current dose distribution obtained for the optimal configuration sample.

According to one embodiment, segmentation is performed by a previously trained convolutional neural network configured to receive as input the 3D medical image and provide as output a semantic 3D segmentation map.

According to one embodiment, the system further comprises an editing interface for clinicians to review and modify intermediate results before finalizing deliverable radiotherapy plan.

According to one embodiment, the at least one processor is further configured to interface with a hospital data management system to automatically collect said at least one 3D medical image, dose prescription parameters for said patient and/or clinical configurable parameters.

According to one embodiment, the at least one processor is further configured to interface with a hospital data management system to automatically collect said at least one 3D medical image (also called in the present description image data) and dose prescription parameters for said patient. Advantageously, with the integration with hospital data management systems, the present embodiment streamlines the entire planning process, reducing the time and effort required from clinicians and physicists, while also improving the consistency and accuracy of the resulting treatment plans.

This invention thus relates to computer-implemented solution (i.e., apparatus) for automating radiotherapy planning, comprising:
- a software platform able to operate in on-premise and/or cloud-based infrastructures, said software platform being configured to implement the steps of the method for automatically generating a deliverable radiotherapy plan for a patient according to the embodiments of the present disclosure;
- a deployment module configured to enable scalable installation of said software platform across different computational mediums;
- a radiotherapy planning engine that automates treatment planning by processing patient data, anatomical models, and treatment protocols.

Advantageously, this software platform is medium-agnostic, allowing for seamless transition and scalability between on-premise and cloud-based deployments, ensuring consistent performance and accessibility in both environments.

According to one embodiment,, the deployment module is configured to dynamically adjusts resource allocation and computational power based on the operational environment, further comprising:
- a cloud integration interface for elastic scaling of resources in cloud-based deployments;
- an on-premise resource manager that configures local computational infrastructure for efficient data processing and data communication;
wherein the system provides continuous automated radiotherapy planning by adapting the infrastructure to the operational demands of the chosen deployment medium.

In addition, the disclosure relates to a computer program comprising software code adapted to perform a method for automatically generating a deliverable radiotherapy plan for a patient or a method for training compliant with any of the above execution modes when the program is executed by a processor.

The present disclosure further pertains to a non-transitory program storage device, readable by a computer, tangibly embodying a program of instructions executable by the computer to perform a method for automatically generating a deliverable radiotherapy plan for a patient or a method for training, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:
The terms "**adapted**" and "**configured**" are used in the present disclosure as broadly encompassing initial configuration, later adaptation or complementation of the present device, or any combination thereof alike, whether effected through material or software means (including firmware).

The term "**processor**" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

"**Machine learning** (**ML**)" designates in a traditional way computer algorithms improving automatically through experience, on the ground of training data enabling to adjust parameters of computer models through gap reductions between expected outputs extracted from the training data and evaluated outputs computed by the computer models.

A "**hyper-parameter**" presently means a parameter used to carry out an upstream control of a model construction, such as a remembering-forgetting balance in sample selection or a width of a time window, by contrast with a parameter of a model itself, which depends on specific situations. In ML applications, hyper-parameters are used to control the learning process.

"**Datasets**" are collections of data used to build an ML mathematical model, so as to make data-driven predictions or decisions. In "**supervised learning**" (i.e. inferring functions from known input-output examples in the form of labelled training data), three types of ML datasets (also designated as ML sets) are typically dedicated to three respective kinds of tasks: "**training**", i.e. fitting the parameters, "validation", i.e. tuning ML hyperparameters (which are parameters used to control the learning process), and "**testing**", i.e. checking independently of a training dataset exploited for building a mathematical model that the latter model provides satisfying results.

A "**neural network** (**NN**)" designates a category of ML comprising nodes (called "**neurons**"), and connections between neurons modeled by "**weights**". For each neuron, an output is given in function of an input or a set of inputs by an "**activation function**". Neurons are generally organized into multiple "**layers**", so that neurons of one layer connect only to neurons of the immediately preceding and immediately following layers.

The above ML definitions are compliant with their usual meaning, and can be completed with numerous associated features and properties, and definitions of related numerical objects, well known to a person skilled in the ML field. Additional terms will be defined, specified or commented wherever useful throughout the following description.

"**Organ at risk** (**OAR**)" refers to a critical anatomical structure or tissue that, due to its location relative to the target treatment area (i.e., target volume), is susceptible to unintended radiation exposure during radiotherapy. The OAR is identified and delineated during treatment planning to minimize radiation-induced damage and ensure that the radiation dose received by the OAR does not exceed established safety thresholds, thereby preserving its function and reducing the risk of radiation-induced side effects.

"**Target volume**" refers to the specific anatomical region identified for receiving therapeutic radiation. This volume encompasses the tumor or malignant tissue intended for treatment and may include surrounding tissues that have a high likelihood of containing microscopic disease. The Target Volume is precisely delineated during the planning process to ensure that the prescribed radiation dose is accurately delivered to the intended area while minimizing exposure to surrounding healthy tissues and Organs at Risk (OARs).

"**Dose-Volume Histogram (DVH) limit**" refers to a predefined constraint or threshold applied to the DVH, which represents the distribution of radiation dose across a specified volume of tissue. The DVH limit is designed to ensure that the prescribed radiation dose is administered within acceptable safety and efficacy parameters. For target volumes, the DVH limit ensures that a sufficient dose is delivered to the tumor, thereby maximizing therapeutic effectiveness. For Organs at Risk (OARs), the DVH limit constrains the radiation dose to mitigate the risk of adverse effects on surrounding healthy tissues.

**"VMAT (Volumetric Modulated Arc Therapy)"** refers to a method of delivering radiation therapy, wherein the radiation dose is continuously administered while the treatment apparatus, specifically a linear accelerator, rotates around the patient along a defined arc. The method is characterized by the simultaneous modulation of three parameters during the rotation: the angle of rotation, the shape of the radiation beam (adjusted through a multi-leaf collimator), and the intensity of the radiation dose. This continuous modulation allows for precise targeting of the tumor while minimizing radiation exposure to adjacent healthy tissues. The method further enables reduced treatment times and improved dose distribution, providing enhanced therapeutic outcomes compared to fixed-beam radiation therapies.

**"Control points"** refers to specific reference points that define the parameters of the radiation beam as the treatment machine (linear accelerator) delivers the dose in an arc around the patient. At each control point, the system defines and records various parameters of the treatment delivery, including the position and shape of the multi-leaf collimator (MLC), the dose rate (the speed at which radiation is delivered), and the number of monitor units (MUs) delivered (MUs are a measure of the amount of radiation delivered), the gantry angle, and sometimes the couch position.

**"Dynamic Arc Optimization** (**DAO**)**"** refers to the is a process used in VMAT to optimize the treatment plan by adjusting the control points to achieve the desired dose distribution. DAO algorithms optimize the position and shape of the MLC at each control point, the dose rate, and other variables to improve the plan's effectiveness.

A "**dose engine"** refers to a computational system or software component used to calculate the radiation dose delivered to a patient during treatment. It models how radiation interacts with tissues to determine the precise dose distribution across the target (tumor) and surrounding healthy tissues (organs at risk).

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a block diagram representing schematically a particular mode of a system for generating a deliverable radiotherapy plan for a patient, compliant with the present disclosure;
**Figure 2** is a flow chart showing successive steps executed with the system of figure 1;
**Figure 3** diagrammatically shows an apparatus integrating the functions of the system of figure 1.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

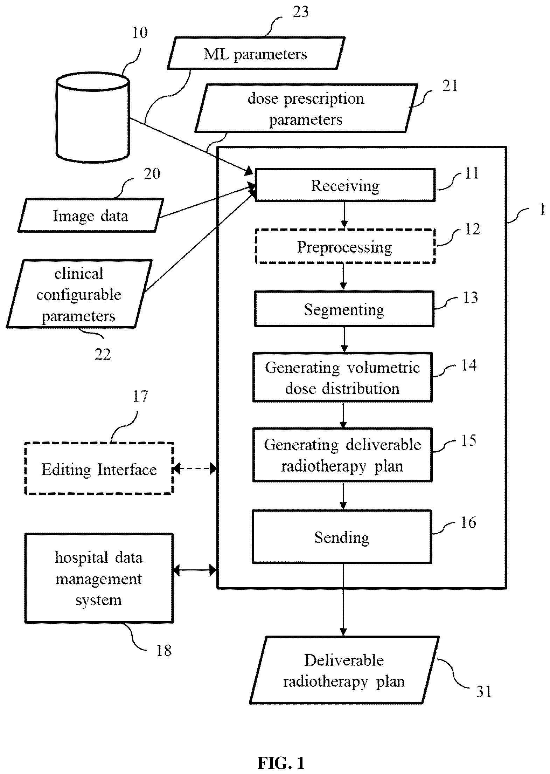

The present disclosure will be described in reference to a particular functional embodiment of a system 1 for automated radiotherapy planning, as illustrated on **Figure 1****.**

The system 1 is adapted to produce a deliverable radiotherapy plan 31 for a patient, to be delivered by a predefined radiotherapy delivery system. With the term "deliverable radiotherapy plan" it is referred to a finalized treatment plan in radiotherapy that has been carefully designed, optimized, and verified to meet all clinical and technical requirements, ensuring that it can be safely and effectively administered to a patient using a specific (predefined) radiotherapy delivery system.

The system 1 for generating may be associated with a device 6 for training suited to setting models parameters of the system 1 in a proper way, represented on **Figure 1****,** which will be subsequently described.

Though the presently described system 1 and device 6 are versatile and provided with several functions that can be carried out alternatively or in any cumulative way, other implementations within the scope of the present disclosure include devices having only parts of the present functionalities.

Each of the system 1 and device 6 is advantageously an apparatus, or a physical part of an apparatus, designed, configured and/or adapted for performing the mentioned functions and produce the mentioned effects or results. In alternative implementations, any of the system 1 and device 6is embodied as a set of apparatus or physical parts of apparatus, whether grouped in a same machine or in different, possibly remote, machines. The system 1 and/or device 6 may e.g. have functions distributed over a cloud infrastructure and be available to users as a cloud-based service, or have remote functions accessible through an API.

The system 1 for generating a deliverable radiotherapy plan 31 and the device 6 for training may be integrated in a same apparatus or set of apparatus and intended to same users. In other implementations, the structure of device 6 may be completely independent of the structure of system 1 and may be provided for other users.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device 6.

The device 1 comprises a **module 11 for receiving the image data 20,** dose prescription parameters 21 and clinical configurable parameters 22, as well as ML (machine learning) parameters 23 stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the ML parameters 20 have been previously generated by an apparatus including the device 6 for training. Alternatively, the ML parameters 20 are received from a communication network.

The image data 20 (i.e., at least one 3D medical image) may comprise at least one 3D image acquired from medical imaging modality, said medical imaging modality being a Computed Tomography (CT) scanner, also called "planning CT image". The image data 20 may as well comprise an identifier associated to the specific CT scanner used to acquire the 3D image(s). Image data 20 may further comprise at least one MRI image (i.e., medical imaging modality being Magnetic Resonance Imaging) comprising a representation of a target volume to be treated, that may be notably used for more accurate identification of the structures of interest (i.e., target volume et OAR). The at least one 3D image 20 comprises a representation of one or more target volumes to treat with radiotherapy and the one or more OAR that should be speared as much as possible during the treatment.

The dose prescription parameters 21 may comprise a prescribed dose for the target volume(s) and Dose-Volume Histogram (DVH) limit. Notably the dose prescription parameters 21 may comprise at least one maximum dose prescription and minimum dose prescription for the target volume and at least one minimum dose prescription, and optionally one maximum dose prescription, for each of the one or more organs at risks. In one example, dose prescription parameters 21 may be extracted from a dose prescription that may comprises information such as localized prostate (i.e., target volume) 60 Gy to be administered in 20 fractions, or prostate (i.e., first target volume) with a dose of 80 Gy combined with lymph nodes (i.e., second target volume) with a dose of 56Gy of a Simultaneous Integrated Boost (SIB) to be administered in 40 fractions. The maximum and minimum doses that could be delivered to target volume may be than extracted using predefined rules, based on medical guidelines. In the same way, the limits, meaning the maximum doses that could be delivered to each OAR, may be obtained from using predefined rules, at least based on medical guidelines. Indeed, clinical guidelines, such as those published by the Radiation Therapy Oncology Group (RTOG), the American Association of Physicists in Medicine (AAPM), or other international bodies, provide dose-volume constraints for OARs. These constraints specify the maximum dose that should be delivered to a certain percentage of the OAR's volume (e.g., V20 < 30%, where V20 is the volume of the organ receiving 20 Gy or more). Other information, such as empirical data, and patient-specific factors may also be considered. In the context of the present invention, all different methods to establish the maximum/minimum doses for OAR or target volumes known by the person of the art may be used.

According to one embodiment, the clinical configurable parameters 22 include at least one of the following: a selection of a linear accelerator (i.e., predefined radiotherapy delivery system) to which the deliverable radiotherapy plan 31 will be targeted (provided the machine has commissioning data available for the system 1); information on the CT scanner on which the planning CT image was acquired and its associated HU-to-ED calibration curve; specification override values for regions in the CT scan that may be out of calibration for the HU-to-ED calibration curve (e.g., prostheses, air pockets or contrast pools, external fixation devices); the number of VMAT arcs of the predefined radiotherapy delivery system to be used for the deliverable radiotherapy plan, along with the gantry angle ranges and collimator angle for each arc, with these hardware settings limited to a set of allowed values.

The system 1 further comprises optionally a module 12 for preprocessing the received image data 20. The module 12 may notably be adapted to standardize the received image data 20 for sake of efficient and reliable processing.

The system 1 may further comprise a module 13 for automatic segmentation of OARs and target volume(s). This module 13 aims at providing an automatic semantic segmentation of organs at risk(s) and target volume(s) respecting specific radiotherapy guidelines for the application of automatic radiation treatment planning. Module 13 may be configured to perform segmentation using a previously trained convolutional neural network configured to receive as input the 3D medical image and provide as output a semantic 3D segmentation map.

The module 13 is configured to implement one or more segmentation neural networks configured to receive as input the 3D image and outputs a 3D volume of the same shape, but with a 4^{th} channel dimension, where each channel represents a given organ (i.e., comprising therefore the organs that may be organs at risk for the different patients), or a target volume (i.e., tumor), and the value in each voxel is the probability that the voxel belongs to said given organ or target volume.

The segmentation neural network(s) follow(s) the broad U-net architecture, where the input data is first sequentially down sampled into feature maps in the encoder and then in the decoder, the features maps are then sequentially up sampled to obtain the final segmentation, leveraging skip connection between the encoder and the decoder. This architecture may be used with both convolutional encoder and decoder or with transformer-like networks.

The segmentation neural network(s) may have been previously trained offline for example by the device 6 for training. Notably the device 6 for training may comprise a module 61 for training this segmentation neural network. To train said segmentation neural network(s), two independent training and tests datasets were set up, made of a plurality of pairs of 3D medical image and contours, each pair being obtained from a different patient to avoid any kind of indirect data leaking. The training set is then split into two independent set: training and validation. The validation set being used to monitor the training and take decisions over performances.

A patch-based training may be applied where crops of a given fixed shape are taken center around the different organs as well as randomly in the 3D volume (i.e., 3D image). In order to improve robustness of the training may be further performed data augmentation on the training set made of random noise, random affine transformations, random contrast and intensity modulation.

The segmentation neural network(s) may be trained with an Adam optimizer, on a loss function that is the sum of a soft dice loss, cross-entropy loss and regularizers such as weight decay. The loss function is monitored overall and per organ on the validation set to ensure non overfitting.

A single network cannot cope efficiently with the number of organs of the human body because of the shape of the output tensors. Therefore, an advantageous strategy of building several segmentation models (i.e., neural networks) may be adopted, wherein each segmentation model is configured to segment only some neighboring organs in the body (i.e., a predefined group of neighboring organs). For example, 15 to 20 segmentation neural networks may be trained and utilized for the segmentation process.

Applying patch-based inference in a sliding window manner over 20 segmentation models for every image can take too long, especially since most of the models are expected to predict no organ in most of the patches.

Module 13 for segmentation may be further configured to implement a fast online inference via coarse localization, when more than one segmentation model is available. Advantageously, the goal of fast localized inference is to decrease the number of patches sent to each segmentation model by choosing which patch to send to which segmentation model.

To perform this fast localized inference, module 13 is configured to run the inference of one segmentation model only where there is a chance to obtain a positive prediction from said segmentation model. To that end, a coarse multi-organ segmentation model is trained (for example by device 6) over low resolution 3D medical images only on a set of 20 large organs spanning the whole body. The coarse multi-organ segmentation model may as well be based on a U-net architecture. The coarse multi-organ segmentation model may be trained with an Adam optimizer, on a loss function that is the sum of a soft dice loss, cross-entropy loss and regularizers such as weight decay.

Every 3D image is then localized at inference time by being first down sampled to the resolution of the coarse multi-organ segmentation model, serving as a localizer, then the down sample 3D image is feed to the coarse multi-organ segmentation model. The bounding box associated to every organ is then defined based on anatomical rules over the bounding boxes predicted by said localizer. These bounding boxes associated to every organ are call herein landmark organs. Those anatomical rules are combined to obtain for each (high-resolution) segmentation model a crop (i.e., a patch) in which to run the inference.

The set of trained segmentation neural networks over organ groups predict for each voxel the organ they are associated with. The fast localized inference makes this process faster by reducing the number of unlikely inferences. However, the different predictions might lead to clinical topological abnormalities, such as non-continuous organs, overlapping organs, non-contiguous organs, holes, several connected components, etc.

To address those abnormal cases, module 13 may be further configured to implement a step of configurable postprocessing, that aims at enforcing those clinical constraints based on the per-organ predictions. As for the localizer's rules, the anatomical rules have been defined by clinical experts and fine-tune or validated on real data.

The localizer's rules positioning every bounding box of every organ with respect to the coarse localizer's detected organs has been defined with clinical experts and refined on real data.

Advantageously, module 13 may be used for different types of cancers as prostate cancer, head and neck cancers, brain tumors, lung cancer, esophageal cancer, rectal cancer, gynecological cancers, breast cancer, pancreatic cancer, and liver cancer, and the like.

The device 1 may further comprise a module 14 for prediction of a volumetric dose distribution that is specific to the received dose prescription parameters 21, but agnostic to the linear accelerator that will be used to deliver the treatment (e.g., VersaHD versus TrueBeam Millenium).

Module 14 is configured to obtain a 3D electronic density distribution from the at least one 3D medical image. This 3D electronic density distribution may be received from a database (i.e., 10) or calculated from the at least one 3D medical image using the HU-to-ED calibration curve associated to the CT-scanner used from acquiring the 3D medical image. The electronic density value represents the density of electrons in a tissue, which determines how much that tissue attenuates X-rays. Advantageously, this distribution is essential for applications like radiotherapy, where precise dose calculations are necessary to target tumors effectively while sparing healthy tissues. The preprocessing pipeline described is also applied here to input CT images given in Hounsfield units along with a calibration curve, 3D electronic density distribution override values, and binary segmentations.

Module 14 is configured to obtain voxel-wise conditioning variables based on said dose prescription parameters 21 and said segmented target volume and one or more segmented organs at risks. The voxel-wise conditioning variables comprises first conditioning variables and second conditioning variables.

Each first conditioning variable is associated to a voxel of the at least one 3D medical image and represents a minimum value of dose that should be delivered during the radiotherapy treatment to the target volume or to one or more organs at risks represented in said voxel. In other words, the first conditioning variables consist of minimum allowed values for each voxel, determined by the contour(s) (i.e., segmented target volume and one or more segmented organs at risks) in which the voxel is included, and the dose prescription parameters 21 (e.g., minimum prescribed dose, DVH limits) associated with that structure (i.e., target volume or organ at risk).

In the same way, each second conditioning variable is associated to a voxel of the at least one 3D medical image and represents a maximum value of dose to be delivered to the target volume or to one or more organs at risks represented in said voxel. In other words, the first conditioning variables consist of minimum allowed values for each voxel, determined by the contour(s) in which the voxel is included, and the dose prescription parameters 21 (e.g., maximum prescribed dose, DVH limits) associated with that structure (i.e., target volume or organ at risk).

The module 14 may further be configured to scale the first conditioning variables and the second conditioning variables using the maximum dose prescription having the greatest magnitude among the dose prescription parameters. magnitude (e.g., 80 Gy for a prostate 80 Gy plus lymph nodes 56 Gy with a Simultaneous Integrated Boost -SIB-prescription).

Module 14 is configured to feed said 3D electronic density distribution, said first conditioning variables and said second conditioning variables to a previously trained dose prediction model configured to provide as output a prediction of the 3D volumetric dose distribution.

The dose prediction model is a convolutional neural network. In one example, this neural network follows the broad U-net architecture, where the input data is first sequentially down sampled into feature maps in the encoder and then in the decoder, the features maps are then sequentially up sampled to obtain the final volumetric dose distribution, leveraging skip connection between the encoder and the decoder. This architecture can be used with both convolutional encoder and decoder or with transformer-like networks.

To obtain the trained dose prediction model, a convolutional neural network (notably a U-net) may be trained using L2 reconstruction loss function, optionally combined with a GAN loss to encourage the hypothetical dose distributions to be clinically realistic. The trained dose prediction model is obtained by training said convolutional neural network (notably a U-net) using a dose training dataset. The dose training dataset may comprise multiple dose training samples each associated to a subject. Each dose training sample, comprises for one subject and an associated prescribed dose, a 3D electronic density distribution (obtained from a 3D CT image), voxel-wise conditioning variables (i.e., first conditioning variables and said second conditioning variables that may obtained as described above for module 14) and a dose map (desired volumetric dose distribution), so that each dose training sample comprises an input-output pairs for each prescription in the dose. The subjects are chosen so to have a large variety of different prescriptions (e.g., hypofractionated prostate, normofractionated prostate, prostate plus seminal vesciles SIB, prostate plus lymph nodes SIB, etc.).

In one embodiment, the voxel-wise conditioning variables and the dose maps, of each dose training sample, are scaled by the maximum dose prescription comprising the greatest magnitude among the dose prescription parameters associated to the corresponding prescribed dose for the subject. Advantageously, as both the input dose conditioning channels (i.e., first conditioning variables and second conditioning variables) and output volumetric dose distributions are scaled, the trained dose prediction model is agnostic to the prescribed dose level, and provided sufficient examples of input-output pairs for each prescription in the dose training dataset, a single dose prediction model learns to distinctly predict different prescriptions (e.g., hypofractionated prostate, normofractionated prostate, prostate plus seminal vesciles SIB, prostate plus lymph nodes SIB, etc.).

According to one embodiment, when the input conditioning channels are scaled, and the dose prediction model is trained on scaled inputs, the volumetric dose distribution obtained from the dose prediction model is unscaled using said maximum dose prescription to obtain clinically viable 3D dose volumes. Module 14 may be also configured, for each prescription, to apply a renormalization heuristic that further multiplies the predicted volumetric dose distribution by a scalar factor to have clinically desirable properties (e.g., planned treatment volume -PTV- coverage meeting clinical constraints) prior to using this hypothetical dose (i.e; volumetric dose distribution) to drive a conventional inverse treatment planning procedure.

The system 1 may further comprise an automated treatment plan generation **module 15** configured to construct a deliverable treatment plan by setting up a conventional VMAT direct aperture optimization (DAO) problem with constraints on the optimization variables that are specific to the targeted linear accelerator; furthermore, this classical optimization approach is guided by the predicted volumetric dose distribution generated by module 14 as a strong prior by which to regularize the optimization.

Module 15 is configured to receive the clinical configurable parameters 22 and use them to preprocess the input data (i.e., volumetric dose distribution, segmented target volume and one or more segmented organs at risks and said at least one 3D medical image 20) and set up an optimization problem that includes constraints and penalties on mechanical movement and hardware positions (i.e., gantry movements and positions).

A unique module 15 may be used across all prescriptions and all anatomies. Indeed, module 15 may be configured define "auxiliary" or secondary structures using templates that handle prescription-dependent behavior. The templates contain recipes for the construction of "auxiliary" or secondary structures from the available primary structures (i.e., segmented target volume and one or more segmented organs at risks). Said "auxiliary" or secondary structures may be for example an annular expansion of the PTV, or differences of PTVs and OARs, or other Boolean operations on regions of interest contours. Auxiliary/secondary structures are commonly used in treatment planning, and often created manually or partially automated on a clinic-specific basis using scripts or macros. Module 15 may be configured to define these structures in fully automated manner. The use of these additional structures helps to reduce dose dumping in non-contoured anatomical regions, including reduction of entrance dose near the skin and of random hotspots throughout the body, and generally encourage fast dose drop-off outside PTV structure.

Module 15 is configured to (approximately) project the predicted volumetric dose distribution onto the set of doses corresponding to machine-deliverable plans for the specified linear accelerator. More in detail, the module 15 may be configured to first define a first objective function on the base of said predicted volumetric dose distribution and said clinical configurable parameters 22. The first objective function is defined to comprise at least one, preferably multiples, weighted voxel-wise penalties on deviations from said predicted volumetric dose distribution. These weighted voxel-wise penalties may be, for example, at least one of the following: weighted voxel-wise penalties on deviations from the hypothetical dose distribution, weighted voxel-wise penalties on over- or under-dosing ROIs (regions of interest) relative to certain clinically relevant thresholds, weighted penalties to discourage overly complex MLC aperture shapes and MLC leaf travel patterns, and yet others to discourage variance across control points of the delivered monitor units.

Module 15 may be configured to perform a second step consisting in defining an initial set of configuration samples, each configuration sample comprising at least a control point, a MLC aperture, a dose rate, a number of delivered monitor units and a gantry angle. The third step that may be implement is the initialization of the (direct aperture) optimization algorithm to generate a current set of configuration samples, starting from said initial set of configuration samples. Module 15 may than, using an optimization algorithm, perform iterative optimization steps to obtain an optimal configuration sample. Notably at each iteration i the optimization algorithm iteratively calculates an *i^{th}* current dose distribution resulting from the *i^{th}* current set of configuration samples and modifies the current set of configuration samples in order to minimize the first objective function (obtaining the new *i+1^{th}* current set of configuration samples to be used in the next iteration to calculate the *i+1^{th}* current dose distribution). These steps are repeated until convergence. In other words. According to one example, the overall optimization problem is solved multiple times at increased angular resolution of the VMAT control points under consideration with solutions of each phase forwarded as initial guesses to the subsequent phase (i.e., iteration).

According to one embodiment, the optimization algorithm is based on a Root Mean Square Propagation (RSMProp) algorithm modified to ensure hard constraints on decision variables by way of iteration-dependent augmentation of first objective function terms related to constraints on clinical configurable parameters 22 associated to said predefined radiotherapy delivery system, which may notably be mechanical constraints on the linear accelerator. In one example, the optimization loop of the modified Root Mean Square Propagation (RSMProp) algorithm is implemented on GPU.

For the VMAT DAO problem to advantageously converge to a high-quality solution with a clinically acceptable dose, it needs to be initialized with good initial aperture shapes. Therefore, the initial set of configuration samples may comprise an initial MLC position (i.e., MLC aperture) for each control point that is calculated using a customized column generation method configured to evaluate the impact of MLC positions over all the control points of the initial set of configuration samples.

According to one example, this customized column generation method further comprises two heuristic steps of resetting a third of the set of selected apertures (from the configuration samples), a first time resetting the least recently selected apertures and a second time taking a randomly chosen subset, as both steps advantageously improve the quality of the initial set of configuration samples and the subsequent VMAT DAO solutions.

After the VMAT DAO problem is solved, the module 15 is further configured to use a dose engine to obtain a simulated volumetric dose distribution corresponding to the obtained optimal configuration sample and then generate said deliverable radiotherapy plan 31 by minimizing a second objective function obtained using said optimization algorithm. Said second objective function may be defined as the first objective function further comprising a term that penalizes deviation between said simulated volumetric dose distribution and the current dose distribution obtained for the optimal configuration sample. In other words, a final heuristic pass of the VMAT DAO is performed to advantageously re-minimize the problem including a term that penalizes deviation between the approximate dose obtained during optimization and the high-fidelity dose obtained during simulation. Empirically, this step minimizes the losses to plan quality that can appear when the optimized radiotherapy plan is simulated by a dose engine.

Examples of dose engines adapted for VMAT are the Eclipse Anisotropic Analytical Algorithm (AAA) or the Monaco Monte Carlo Algorithm from Elekta, which are widely used in VMAT treatment planning, however any other dose engine adapted for VMAT known by the person skilled in the art may be used for the implementation of the present step.

The result of this final polishing phase may be then stored as a radiotherapy plan (RTPLAN), for which the associated dose is simulated in the dose engine and stored as a radiotherapy dose. Module 15 may be further configured to scaled jointly by a scalar multiplicative factor the MUs of the former and the MUs of the latter to advantageously ensure good tumor coverage; the exact definition of "good coverage" is prescription-dependent and comes from a DVH constraint on a PTV or CTV structure. The final generated RTPLAN is now deliverable, in that it meets hardware constraints for the predefined radiotherapy delivery system, and thanks to the optimization's problem structure and guidance from the hypothetical dose distribution, highly likely to pass plan QA and dosimetry evaluation by a clinician.

According to one example, the module 15 performs the following stages:
(1) use customized column generation algorithm to initialize the MCL apertures (i.e., MCL apertures shapes);
(2) run until convergence (2a) the direct aperture optimization algorithm (DAO) to optimize the aperture shapes and intensities for control points every 8 degrees, initialized with column generation algorithm results, run for a fixed number of iterations (2b) DAO again for control points at every 4 degrees, initialized with results from (2a) (copied for existing control points, interpolated for new control points), again run for a fixed number of iterations (2c) DAO with control points at every 2 degrees, initialized with the results from (2b);
(3) for a fixed number of iterations calculate the high-fidelity dose (i.e., simulated volumetric dose distribution) using plan emitted by (2c) (i.e., optimal configuration sample);
(4) the result is used to determine the dose discrepancy between the high-fidelity dose calculation and the DAO optimization's internal representation of the optimized dose (4a) DAO at 4 degrees resolution using an additional penalty term designed to minimize the dose discrepancy (4b) DAO at 2 degrees resolution using the same penalty term; and
(5) final dose calculation with the dose engine using the plan emitted by (4b).

The system 1 may be further configured to interface with a hospital data management system 18. This may notably allow to automatically collect said at least one 3D medical image 20, dose prescription parameters 21 for said patient and/or clinical configurable parameters 22, but also to send the deliverable radiotherapy plan 31 to a predefined radiotherapy delivery system for final verification and execution.

The integration of the full auto-planification pipeline (i.e., modules 11 to module 15) to the hospital data management system 18 may be made through different modules: a DICOM gateway, a data reception module, a workflow automation module, editing interface and/or plan DICOM export.

Notably, all the modules described herein may be integrated into a software platform, which may be cloud based, said software platform communicating with at least one sever.

The preferred communication protocol on the hospital premises is DICOM, that stands for Digital Imaging and Communications in Medicine and which encompasses a communication protocol and a file format. In other to configure the DICOM **gateway** to receive and handle DICOM files, a DICOM node (application entity) is installed on the hospital premises, that is responsible for receiving DICOM files, aggregating them into DICOM series and sending them over a secure HTTPS protocol to the software platform for processing. This DICOM gateway can be seen from other Dicom applications on the same network such as acquisition hardware or treatment planning system. The DICOM gateway module is entry point to the present automated radiotherapy planning system, it communicates via HTTPS to the software platform and is uniquely identified via secured credentials.

The DICOM gateway may be installed as a Windows service or as a Linux daemon. Once installed, it exposes one or several application entity titles that can be used to forward the series to the right processing pipeline.

The DICOM gateway may be configure locally or remotely to set up the communication layers with other DICOM applications and to cope with different scenarios in terms of data aggregation, data format, de-identification, re-identification, data upload, etc., depending on the requirements of the different institutes. These different steps are important because in the case of data aggregation, there is no robust way to know when all the slices of a 3D volume have been received. This may vary from one center to the other based on internal protocols and network setup. In terms of data privacy, DICOM header might contain personal data that cannot be sent outside of the hospital network, but that needs to be sent back to the treatment planning system.

The DICOM gateway first forwards DICOM series such as images, RT plan, RT dose or RT Struct to our backend via a secure and authenticated session. Then the data reception module pushes those data onto an object storage and referenced via unique identifiers (UIDs). On the server side, the DICOM data is parsed and DICOM headers are indexed so that they can later be queried upon. Already indexed DICOM files may be not re-indexed to optimize for storage and DB space in case of several DICOM pushes of the same series.

Once the DICOM gateway has pushed all the files needed for OAR segmentation or plan optimization, a workflow automation module triggers the workflow that will automatically execute the steps of the method for automatically generating a deliverable radiotherapy plan, notably:
- segment the said target volume and one or more organs at risks;
- predict the 3D volumetric dose distribution;
- optimize the deliverable radiotherapy plan 31;
- optionally, indexation of Radiotherapy Plan (i.e., file that contains detailed information about the radiotherapy treatment plan, including dose distribution, treatment beams, angles, and settings), Radiotherapy Structure Set (RTSS), Radiotherapy Dose (i.e., file contains the calculated or planned radiation dose distribution within the patient's body) into the data storage.

Advantageously the above described modules for integration to the hospital data management system 18 allow to compose automated workflow based on core components (data management, identity and access management, computational resource allocation and scheduling, product configurations, versioning of technologies, slicer, event log) and specific technologies or operators (dose prediction, organ segmentation, plan optimization, deformable registration, dose engine, radiomics extraction, etc.) by product components responsible for enforcing the business logic (workflow orchestration, data retention, clinical safe guards, etc.).

The software may either work in a fully automated pipeline or propose an editing interface 17 configured to allow to review and modify intermediate results before finalizing deliverable radiotherapy plan 31. This editing interface for clinicians 17 may be notably configured to correct the contours prior to the plan's optimization. The editing interface 17 may comprise a 3D slicer with edition tools such as brush and eraser of adjustable size.

The editing interface for clinicians 17 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

The produced DICOM files may be stored on the server side. The DICOM gateway may poll the server to get back the results whenever ready. Those files may be downloaded locally on the client side (e.g., hospital or clinic), where the gateway is installed, re-identified in case of de-identification of patient personal data and pushed to the pre-defined application entity of the hospital's network.

In case of failure, the gateway may also retrieve the error code and produce a DICOM file that contains the error code and description, so that the caller of the computation may be aware of the error and act depending on the error, for example by triggering another computation or changing the configuration of the computation.

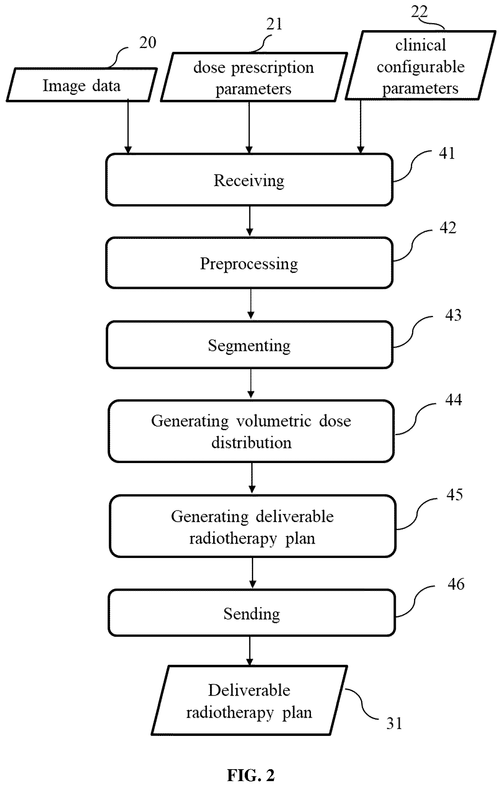

In its automatic actions, the system 1 may for example execute the following process (**Figure 2**):
- receiving the image data 20, dose prescription parameters 21 and clinical configurable parameters 22 (step 41),
- preprocessing the image data 20 (step 42),
- segment said target volume and one or more organs at risks in the 3D medical image, obtaining a segmented target volume and one or more segmented organs at risks (step 43),
- generate a prediction of a 3D volumetric dose distribution based on the 3D medical image, the segmented target volume and one or more segmented organs at risks, and by applying voxel-wise conditioning variables based on said dose prescription parameters 21 (step 44)
- generate said deliverable radiotherapy plan 31 with an optimization algorithm using said volumetric dose distribution, said clinical configurable parameters 22, segmented target volume and one or more segmented organs at risks and said at least one 3D medical image 20 (step 45),
- send the deliverable radiotherapy plan 31 to a predefined radiotherapy delivery system for final verification and execution (step 46).

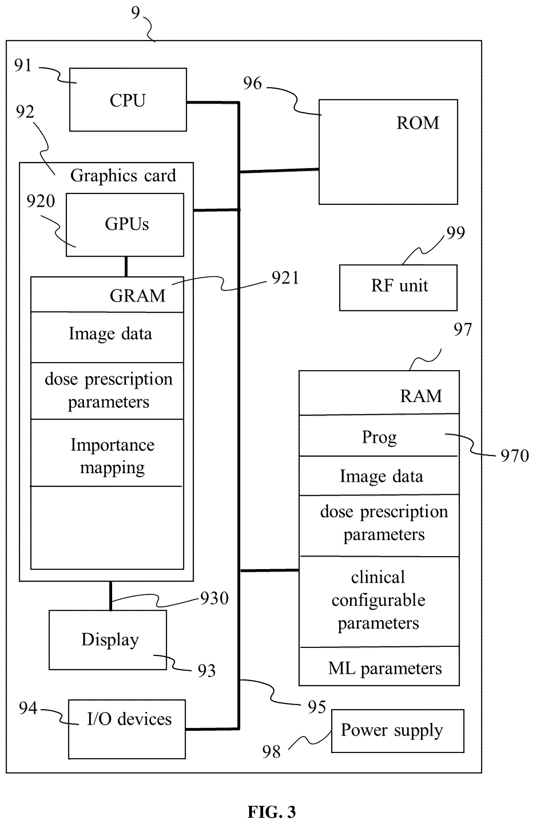

A particular apparatus 9, visible on **Figure 3**, is embodying the system 1 described above. It corresponds for example to a workstation, a laptop, a tablet, a smartphone, or a head-mounted display (HMD).

That apparatus 9 is suited to for generating a deliverable radiotherapy plan 31 for a patient. It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the system 1. In addition, the system 1 may be implemented differently than a standalone software, and an apparatus or set of apparatus comprising only parts of the apparatus 9 may be exploited through an API call or via a cloud interface.

The present invention relates as well to computer-implemented solution (i.e., apparatus) for automating radiotherapy planning, comprising:
- a software platform able to operate in on-premise and/or cloud-based infrastructures, said software platform being configured to implement the steps of the method for automatically generating a deliverable radiotherapy plan for a patient according to the embodiments of the present disclosure;
- a deployment module configured to enable scalable installation of said software platform across different computational mediums;
- a radiotherapy planning engine that automates treatment planning by processing patient data, anatomical models, and treatment protocols.

According to one embodiment, the deployment module is configured to dynamically adjusts resource allocation and computational power based on the operational environment, further comprising:
- a cloud integration interface for elastic scaling of resources in cloud-based deployments;
- an on-premise resource manager that configures local computational infrastructure for efficient data processing and data communication;
wherein the system provides continuous automated radiotherapy planning by adapting the infrastructure to the operational demands of the chosen deployment medium.

## Claims

1. An automated radiotherapy planning system for generating a deliverable radiotherapy plan (31) for a patient, to be delivered by a predefined radiotherapy delivery system, wherein the system comprises:
- at least one input configured to receive:
• at least one 3D medical image (20) obtained from a medical imaging modality, said at least one 3D medical image comprising a representation of a target volume to be treated, with said deliverable radiotherapy plan, and one or more organs at risks of said patient;
• dose prescription parameters (21) for said patient, said dose prescription parameters (21) comprising at least one maximum dose prescription and minimum dose prescription for the target volume and at least one maximum dose prescription for each of the one or more organs at risks;
• clinical configurable parameters (22) associated to said predefined radiotherapy delivery system and said medical imaging modality;
- at least one processor configured to:
• segment said target volume and one or more organs at risks in the 3D medical image, obtaining a segmented target volume and one or more segmented organs at risks;
• generate a prediction of a 3D volumetric dose distribution based on the 3D medical image, the segmented target volume and one or more segmented organs at risks, and by applying voxel-wise conditioning variables based on said dose prescription parameters (21);
• generate said deliverable radiotherapy plan (31) with an optimization algorithm using said volumetric dose distribution, said clinical configurable parameters (22), segmented target volume and one or more segmented organs at risks and said at least one 3D medical image (20);
• send the deliverable radiotherapy plan (31) to said predefined radiotherapy delivery system for final verification and execution.

2. The system according to claim **1**, wherein generate a prediction of a volumetric dose distribution comprises:
- obtain a 3D electronic density distribution from the at least one 3D medical image and a clinical configurable parameter associated to said medical imaging modality;
- obtain voxel-wise conditioning variables based on said dose prescription parameters (21) and said segmented target volume and one or more segmented organs at risks, wherein said voxel-wise conditioning variables comprises first conditioning variables and second conditioning variables,
each first conditioning variable being associated to a voxel of the at least one 3D medical image and representing a minimum value of dose to be delivered to the target volume or to one or more organs at risks represented in said voxel; each second conditioning variable being associated to a voxel of the at least one 3D medical image and representing a maximum value of dose to be delivered to the target volume or to one or more organs at risks represented in said voxel;
- feed said 3D electronic density distribution, said first conditioning variables and said second conditioning variables to a previously trained dose prediction model configured to provide as output the predicted volumetric dose distribution.

3. The system according to claim **2**, wherein the first conditioning variables and the second conditioning variables being scaled by the maximum dose prescription comprising the greatest magnitude among the dose prescription parameters (21); and wherein the predicted volumetric dose distribution obtained from the dose prediction model is unscaled using said maximum dose prescription.

4. The system according to any one of claims **1** to **3**, wherein the dose prediction model architecture is a convolutional neural network, preferably a U-net.

5. The system according to any one of claims **1** to **4**, wherein generate said deliverable radiotherapy plan (31) with an optimization algorithm comprises:
- defining a first objective function on the base of said predicted volumetric dose distribution and said clinical configurable parameters (22), said first objective function comprising at least weighted voxel-wise penalties on deviations from said predicted volumetric dose distribution;
- defining an initial set of configuration samples, each configuration sample comprising at least a control point, a multi-leaves collimator (MLC) aperture, a dose rate, a number of delivered monitor units and a gantry angle;
- initializing the optimization algorithm to generate, from said initial set of configuration samples, a current set of configuration samples,
- obtaining an optimal configuration sample by performing iterative optimization of said current set of configuration samples, wherein the optimization algorithm iteratively calculates a current dose distribution resulting from the current set of configuration samples and modifies the current set of configuration samples in order to minimize the first objective function, until convergence.

6. The system according to claim **5**, wherein the first objective function further comprises at least one among: weighted penalties associated to over-dosing or under-dosing regions of interests relative to predefined clinically relevant thresholds, weighted penalties to discourage overly complex multi-leaves collimator aperture shapes and multi-leaves collimator leaf travel patterns, and weighted penalties to discourage variance across control points over the number of delivered monitor units.

7. The system according to either one of claims **5** or **6**, wherein the optimization algorithm is based on a Root Mean Square Propagation (RSMProp) algorithm modified to ensure hard constraints on decision variables by way of iteration-dependent augmentation of first objective function terms related to constraints on clinical configurable parameters (22) associated to said predefined radiotherapy delivery system.

8. The system of according to any one of claims **5** to **7**, wherein the initial set of configuration samples comprises an initial multi-leaves collimator (MLC) aperture for each control point that is calculated using a column generation method configured to evaluate impact of multi-leaves collimator (MLC) apertures over all the control points of the initial set of configuration samples.

9. The system according to any one of claims **5** to **8,** wherein generate said deliverable radiotherapy plan (31) with an optimization algorithm further comprises:
- obtain a simulated volumetric dose distribution using a dose engine and the obtained optimal configuration sample;
- generate said deliverable radiotherapy plan (31) by minimizing a second objective function obtained from said current dose distribution using said optimization algorithm, wherein said second objective function is defined as the first objective function further comprising a term that penalizes deviation between said simulated volumetric dose distribution and the current dose distribution obtained for the optimal configuration sample.

10. The system according to any one of claims **5** to **9**, wherein segmentation is performed by a previously trained convolutional neural network configured to receive as input the 3D medical image and provide as output a semantic 3D segmentation map.

11. The system according to any one of claims **5** to **10**, further comprising an editing interface for clinicians to review and modify intermediate results before finalizing said deliverable radiotherapy plan (31).

12. The system according to any one of claims **5** to **10**, wherein the at least one processor is further configured to interface with a hospital data management system (18) to automatically collect said at least one 3D medical image (20), dose prescription parameters (21) for said patient and/or clinical configurable parameters (22).

13. A method for automatically generating a deliverable radiotherapy plan (31) for a patient, to be delivered by a predefined radiotherapy delivery system, wherein the system comprises:
- receiving:
• at least one 3D medical image (20) obtained from a medical imaging modality, said at least one 3D medical image comprising a representation of a target volume to be treated, with said deliverable radiotherapy plan, and one or more organs at risks of said patient;
• dose prescription parameters (21) for said patient, said dose prescription parameters (21) comprising at least one maximum dose prescription and minimum dose prescription for the target volume and at least one maximum dose prescription for each of the one or more organs at risks;
• clinical configurable parameters (22) associated to said predefined radiotherapy delivery system and said medical imaging modality;
- segmenting said target volume and one or more organs at risks in the 3D medical image, obtaining a segmented target volume and one or more segmented organs at risks;
- generating a prediction of a 3D volumetric dose distribution based on the 3D medical image, the segmented target volume and one or more segmented organs at risks, and by applying voxel-wise conditioning variables based on said dose prescription parameters (21);
- generating said deliverable radiotherapy plan (31) with an optimization algorithm using said volumetric dose distribution, said clinical configurable parameters (22), segmented target volume and one or more segmented organs at risks and said at least one 3D medical image (20);
- sending the deliverable radiotherapy plan (31) to said predefined radiotherapy delivery system for final verification and execution.

14. A computer program product comprising instructions which, when the program is executed by a computer, cause the computer to carry out the method of claim **13.**

15. A computer-readable storage medium comprising instructions which, when executed by a computer, cause the computer to carry out the method of claim **13.**
